# EUROPEAN PATENT APPLICATION

(11) **EP 1 437 153 A1**
(43) Date of publication of application: **14.07.2004**
(21) Application number: 02747705.8
(22) Date of filing: 22.07.2002
(51) Int. Cl.: A61M 25/00, A61M 5/158, A61M 5/32

(54) **SELF-RETAINING NEEDLE**

(30) Priority: 20.09.2001 JP 2001287688
(71) Applicant: KABUSHIKI KAISHA TOP, Tokyo 120-0035 (JP)
(72) Inventor: SUZUKI, Takashi, Chiba 270-1164 (JP)
(74) Representative: Fenlon, Christine Lesley
(86) International application number: PCT/JP2002/007382
(87) International publication number: WO 2003/026731

(57) **Abstract**

There is provided an easily operated self-retaining needle capable of surely preventing accidental sticking of an inner needle, and touching to blood, humor or the like adhered to the inner needle. This self-retaining needle comprises an outer needle 2, an outer needle hub 4, an inner needle 3 and an inner needle hub 6. An enveloping member 8 is provided for connecting a support member 7 for supporting the outer needle hub 4 with the inner needle hub 6, and enveloping a front of the inner needle 3. In the enveloping member 8, a front portion 10 and a rear portion 12 are freely folded. When a knife point portion 5 is protruded from a tip of the outer needle 2, the front portion 10 and the rear portion 12 are folded, and the rear portion 12 is housed between the inner needle hub 6 and the front portion 10. When the inner needle 3 is retreated toward the rear of the outer needle hub 4, the front portion 10 and the rear portion 12 are expanded along both side faces of the inner needle 3. When a front portion 23 and a rear portion 24 are expanded along both side faces of the inner needle 3, an enveloping member 22 envelops the inner needle 3 by the front portion 23 and the rear portion 24.

## Description

### Technical Field

The present invention relates to a self-retaining needle provided with an outer needle connected to an infusion device or the like, and an inner needle inserted into the outer needle to introduce the outer needle into a blood vessel or the like.

### Background Art

A self-retaining needle has conventionally been known, which is connected to an infusion device or the like for introducing blood, medicine or the like into a body of a patient. The self-retaining needle includes a hollow outer needle connected to the infusion device or the like, and a hollow inner needle for introducing the outer needle into a blood vessel of the patient. When the inner needle is inserted into the hollow portion of the outer needle, its knife point is protruded from a tip of the outer needle.

The self-retaining needle enables the outer needle fitted around the inner needle to be smoothly introduced into the blood vessel or the like by thrusting the knife point of the inner needle into the blood vessel or the like of the patient. After the introduction of the outer needle into the blood vessel or the like, the inner needle is pulled out, an infusion tube or the like of the infusion device or the like is connected to a hub of the outer needle in place of the inner needle, and then blood transfusion, medicine administration or the like is carried out.

On the other hand, the inner needle that has been pulled out is disposed of. In this case, if the knife point is exposed, medical personnel or a worker engaged in disposal may stick the inner needle into a finger or the like accidentally (abbreviated to accidental sticking, hereinafter). Here, if the patient suffers from an infectious disease such as HIV or acute hepatitis, there is a possibility that the medical personnel or the worker engaged in disposal may be infected with the disease through the inner needle.

Furthermore, blood, humor or the like of the patient is adhered to an outer surface of the inner needle. Thus, if the medical personnel or the worker engaged in disposal accidentally touches the blood, humor or the like, there is also a possibility that he may be infected with the disease.

### Disclosure of the Invention

The present invention was made with the foregoing problems in mind, and it is an object of the invention to provide an easily operated self-retaining needle capable of surely preventing accidental sticking of an inner needle pulled out from a body of a patient for medical personnel or a worker engaged in disposal, and touching to blood, humor or the like adhered to the inner needle.

In order to achieve the object, a self-retaining needle of the present invention comprises a hollow outer needle, an outer needle hub provided in a base of the outer needle, a hollow inner needle having a knife point portion on a tip and a length of the knife point portion protruded from a tip of the outer needle when it is inserted from the outer needle hub into the outer needle, and an inner needle hub provided in a base of the inner needle and positioned at the rear of the outer needle hub when the knife point portion of the inner needle is protruded from the tip of the outer needle, comprises a support member for detachably supporting the outer needle hub in front of the inner needle hub, and an enveloping member for connecting the support member with the inner needle hub and enveloping at least a front of the inner needle when the inner needle is retreated toward the rear of the outer needle hub, wherein a front portion and a rear portion of the enveloping member are connected so as to be freely folded, when the knife point portion of the inner needle is protruded from the tip of the outer needle, the front portion and the rear portion of the enveloping member are folded, so that the rear portion is housed between the inner needle hub and the front portion, and when the inner needle is retreated toward the rear of the outer needle hub, the front portion and the rear portion thereof are expanded along both side faces of the inner needle.

According to the self-retaining needle of the present invention, first, the inner needle is trust into a blood vessel or the like by the knife point portion of the inner needle protruded from the tip of the outer needle, and then the outer needle inserted over the inner needle is introduced into the blood vessel or the like. At this time, in the enveloping member, the front portion and the rear portion are folded at both side faces of the inner needle, and the rear portion is housed between the inner needle hub and the front portion. Thus, the enveloping member is prevented from interfering with the operation of sticking the inner needle into the blood vessel or the like, and introducing the outer needle into the blood vessel or the like,

Then, when the inner needle is retreated toward the rear of the outer needle hub in order to pull out the inner needle and retain the outer needle, the front and rear portions of the enveloping member folded as described above are expanded along both side faces of the inner needle. As a result, at least a front of the inner needle is enveloped by the enveloping member.

Therefore, according to the self-retaining needle of the present invention, it is possible to pull out the inner needle by the simple operation of retreating it after the outer needle hub. Moreover, according to the self-retaining needle of the invention, the above-described operation makes it possible to surely prevent medical personnel or a worker engaged in disposal from accidentally sticking the inner needle, or touching blood, humor or the like adhered on the inner needle.

When the inner needle is thrust into the blood vessel or the like, mainly the front of the inner needle enters a body of a patient, and blood, humor or the like of the patient is adhered to this part. Accordingly, it is only necessary for the enveloping member to be capable of enveloping at least the front of the inner needle including the knife point portion causing a sticking accident. However, in order to further assure the prevention of the medical personnel or the worker engaged in disposal from touching the blood, humor or the like adhered on the inner needle, preferably, the enveloping member envelops the entire inner needle.

Thus, when the inner needle is retreated toward the rear of the outer needle hub, and the front portion and the rear portion are expanded along both side faces of the inner needle, the enveloping member envelops the inner needle by the front and rear portions. According to this constitution, since the enveloping member for connecting the support member with the inner needle hub envelops the inner needle by the entire combination of the front and rear portions, the entire inner needle positioned between the support member and the inner needle hub is enveloped by the enveloping member. Therefore, it is possible to more surely prevent the medical personnel or the worker engaged in disposal from touching the blood, humor or the like adhered on the inner needle.

### Brief Description of the Drawings

FIG. 1 is a partially cutout plan view showing a self-retaining needle according to an embodiment of the present invention.
FIG. 2 is a sectional view taken on line II-II of FIG. 1.
FIG. 3 is an explanatory view of an operation of the self-retaining needle shown in FIG. 1.
FIG. 4 is an explanatory view of an operation of the self-retaining needle shown in FIG. 1.
FIG. 5 is a perspective view showing a state of an inner needle enveloped in the self-retaining needle of FIG. 1 with one side face of the inner needle omitted.
FIGS. 6A and 6B are explanatory views showing protrusion preventing means of the inner needle in the self-retaining needle of FIG. 1.
FIGS. 7A and 7B are explanatory views showing protrusion preventing means of the inner needle in the self-retaining needle of FIG. 1.
FIGS. 8A and 8B are explanatory views showing protrusion preventing means of the inner needle in the self-retaining needle of FIG. 1.
FIGS. 9A and 9B are explanatory views showing protrusion preventing means of the inner needle in the self-retaining needle of FIG. 1.
FIG. 10 is a partially cutout plan view showing a self-retaining needle according to another embodiment of the present invention.
FIG. 11 is a sectional view taken on line XI-XI of FIG. 10.
FIG. 12 is an explanatory view of an operation of the self-retaining needle shown in FIG. 10.
FIG. 13 is a perspective view showing a state of an inner needle enveloped in the self-retaining needle of FIG. 10 with one side face of the inner needle omitted.

### Best Mode for Carrying out the Invention

First, description will be made of a self-retaining needle 1 according to a first embodiment of the present invention.

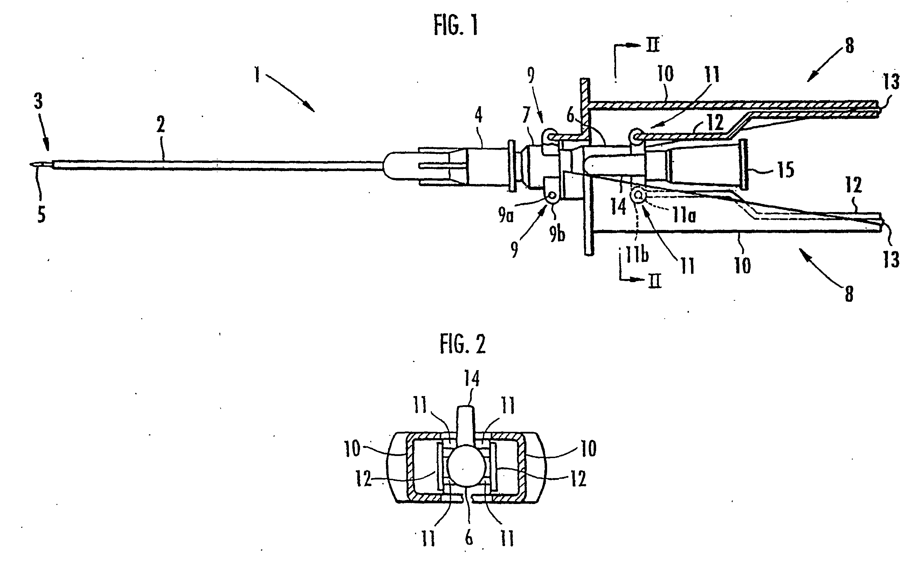

As shown in FIG. 1, the self-retaining needle 1 comprises a hollow outer needle 2, and a hollow inner needle 3 inserted into a hollow portion of the outer needle 2. The outer needle 2 is provided with an outer needle hub 4 on a base. The inner needle 3 is provided with a knife point portion 5 to be protruded from a tip of the outer needle 2 when it is inserted from the outer needle hub 4 into the hollow portion of the outer needle 2, and a inner needle hub 6 positioned at the rear of the outer needle hub 4.

The self-retaining needle 1 also comprises a support member 7 provided before the inner needle hub 6 to detachably support the outer needle hub 4. The support member 7 is fitted in the outer needle hub 6. The self-retaining needle 1 comprises an enveloping member 8 provided to envelop a front part of the inner needle 3 when the support member 7 is connected to the inner hub 6, and the inner needle 3 is retreated toward the rear of the outer needle hub 4.

The enveloping member 8 is arranged to be divided along both side faces of the inner needle hub 6, and composed of a front enveloping portion 10 rotatably attached to a hinge portion 9 protruded in both sides from the support member 7, and a rear portion 12 rotatably attached to a hinge portion 11 protruded in both sides from the inner needle hub 6. The hinge portion 9 is composed of a rotary shaft 9a provided in a tip of the front enveloping portion 10, and a pair of upper and lower bearing surfaces 9b provided in the support member 7 to support the rotary shaft 9a. The hinge portion 11 is composed of a rotary shaft 11a provided in a rear end of the rear portion 12, and a pair of upper and lower bearing surfaces 11b provided in the inner needle hub 6 to support the rotary shaft 11a. The front enveloping portion 10 and the rear portion 12 are connected to each other through a thin portion 13 so as to be folded.

As shown in FIG. 2, the front enveloping portion 10 is formed in a tub shape, where a surface opposite the inner needle hub 6 is opened. The rear portion 12 is formed in a flat plate shape. In a state where the knife point portion 5 of the inner needle 3 is protruded from the tip of the outer needle 2, the front enveloping portion 10 and the rear portion 12 are folded at the thin portion 13. In the above state, the rear portion 12 is arranged between the front enveloping portion 10 and the inner needle hub 6, and housed in the tub-shaped front enveloping portion 10.

A gripper 14 is provided on the inner needle hub 6 to operate the same, and a diffused air tube 15 is connected to a rear end part of the inner needle hub 6. The diffused air tube 15 is provided with a filter inside to ventilate air, and detachably fitted in the rear end part of the inner needle hub 6 through a lure tapered portion at the tip. The filter operates to prevent contamination from the outside, and flowing-out of blood or the like when the inner needle 3 is stuck into the blood vessel or the like.

In place of the diffused air tube 15, the filter may be arranged in the inner needle hub 6 to prevent the flowing-out of the blood or the like.

Next, an operation of the self-retaining needle 1 will be described.

In the self-retaining needle 1, as shown in FIG. 1, the front enveloping portion 10 and the rear portion 12 of the enveloping portion 8 are folded at the thin portion 13, and the rear portion 12 is housed in the tub-shaped front enveloping portion 10. In the self-retaining needle 1, first, in the above state, the knife point portion 5 of the inner needle 3 protruded from the tip of the outer needle 2 is stuck into the blood vessel or the like of the patient. Then, the outer needle 2 fitted around the inner needle 3 is introduced from a rear side of the knife point portion 5 into the blood vessel or the like.

At this time, in the indwelling portion 1, the enveloping member 8 is in the folded state as described above. Thus, the enveloping member 8 is prevented from interfering with the operation of sticking the inner needle 3 into the blood vessel or the like, and introducing the outer needle 2 into the blood vessel or the like.

After the introduction of the outer needle 2 into the blood vessel or the like, the inner needle 3 is pulled out in order to connect an infusion tube or the like of an infusion device or the like to the outer needle hub 4 in place of the inner needle 3. At this time, medical personnel such as a doctor or a nurse grips the gripper 14 provided on the inner needle hub 6 while fixing the outer needle 2 to retain it in its introduced state to the blood vessel or the like, and retreats the inner needle hub 6.

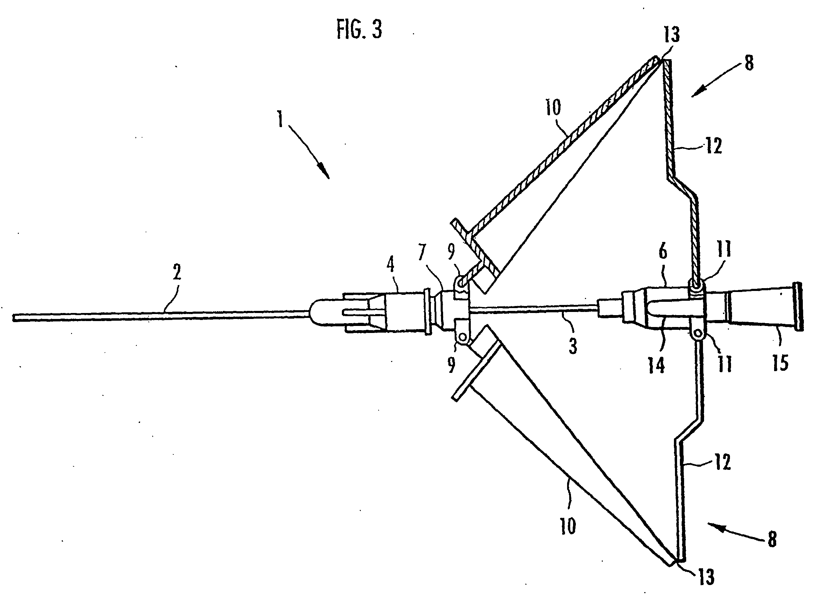

The front enveloping portion 10 of the enveloping member 8 is rotatably attached through the hinge portion 9 to the support member 7, and the rear portion 12 is rotatably attached through the hinge portion 11 to the inner needle hub 6. Accordingly, when the inner needle hub 6 is retreated as described above, following the retreating, the front enveloping portion 10 and the rear portion 12 are rotated toward the outer needle hub 4. As a result, as shown in FIG. 3, the front enveloping portion 10 and the rear portion 12 are thrown out in sides of the inner needle 3.

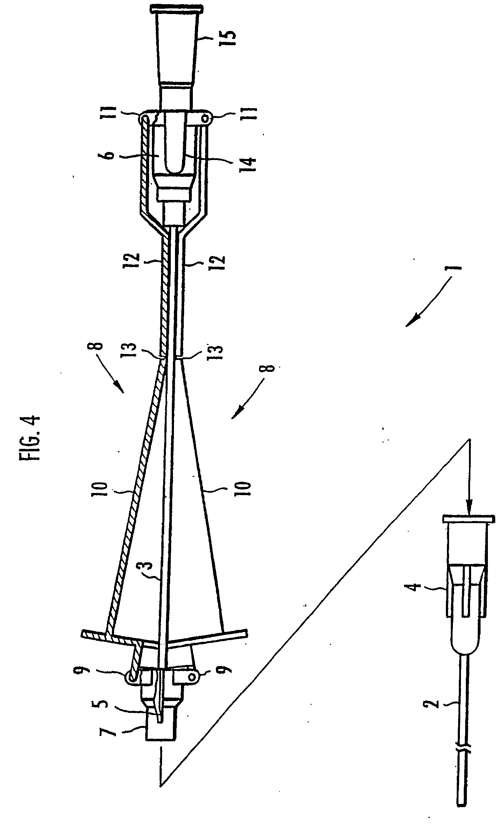

Then, the inner needle hub 6 is further retreated, and the knife point portion 5 of the inner needle 3 is retreated toward the rear of the outer needle hub 4. Then, as shown in FIG. 4, the front enveloping portion 10 and the rear portion 12 are completely expanded along the inner needle 3 on both side faces thereof to be linear.

Thus, by removing the support member 7 from the outer needle hub 4, the inner needle 3 can be disposed of. As described above, after the removal of the support member 7, an infusion tube or the like of an infusion device or the like is connected to the outer needle hub 4.

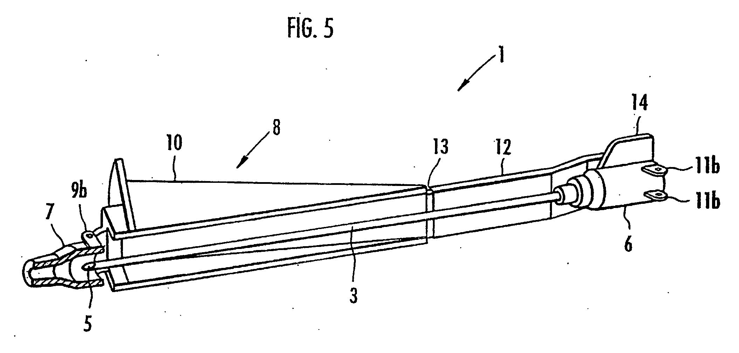

As described above, in the self-retaining needle 1 removed from the outer needle hub 4, the front enveloping portion 10 and the rear portion 12 have been completely expanded. As a result, as shown in FIG. 5, on both sides of the inner needle 3, the open surface of the tub-shaped front enveloping portion 10 faces the inner needle 3, and the front part of the inner needle 3 is enveloped by the front enveloping portions 10 and 10. For explanation, only one front enveloping portion 10 is shown in FIG. 5.

Thus, it is possible to surely prevent the medical personnel or the worker engaged in needle disposal from accidentally sticking the knife point portion 5 of the inner needle 3, or touching the front part of the inner needle 3 on which blood, humor or the like of a patient is adhered.

Also, on a rear portion of the inner needle 3, since the flat plate-shaped rear portion 12 is arranged in both side faces at this time, both upper and lower surfaces are opened only in width slightly larger than a thickness of the inner needle 3. Accordingly, it can be made difficult for the medical personnel or the worker engaged in needle disposal to touch blood, humor or the like adhered on the inner needle 3.

In this case, the self-retaining needle 1 comprises protrusion preventing means provided to prevent the knife point portion 5 of the inner needle 3 retreated into the support member 7 once after the outer needle hub 4 from being protruded from the support member 7.

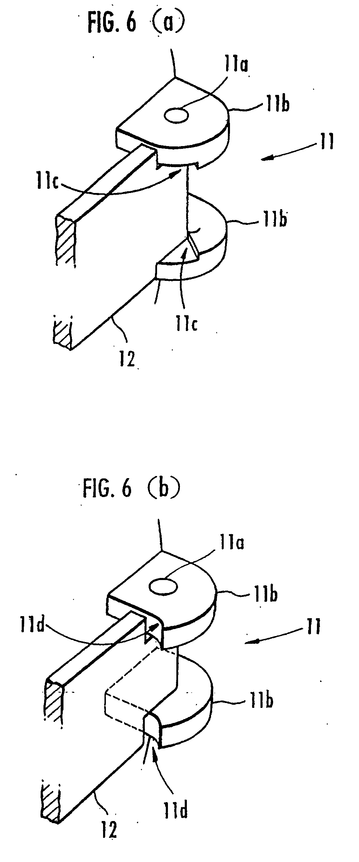

When the protrusion preventing means is provided, for example as shown in FIG. 6A, by using the pair of upper and lower bearing surfaces 11b and 11b as rotation guiding members, parts of the bearing surfaces 11b and 11b are formed in slope shapes so as to be gradually protruded from surfaces. Then, a stepped portion 11c provided on a boundary with the surface of each of the bearing surfaces 11b and 11b on a most protruded part of the sloped portion (slope portion) can be used as the protrusion preventing means.

According to the constitution shown in FIG. 6A, in the state where the front enveloping portion 10 and the rear portion 12 are folded as shown in FIG. 1, the rear portion 12 is positioned on a base (right end of the bearing surface 11b shown in FIG. 6A) of the slope portion. Then, following the operation of laying out the front enveloping portion 10 and the rear portion 12 as shown in FIG. 4 through the state of FIG. 3, the rear portion 12 is guided by the bearing surface 11b, and rotated from right to left in the drawing.

Then, by going over the most protruded part of the slope portion, the rear portion 12 is engaged with the stepped portion 11c. As a result, the rear portion 12 is regulated by the stepped portion 11c, and the knife point portion 5 is prevented from being rotated again in a protruding direction (from left to right in the drawing).

With the bearing surface 11b as a rotation guiding member as shown in FIG. 6B, the protrusion preventing means may be a stepped portion 11d formed from outside to inside on a side face of the bearing surface 11b. According to the constitution shown in FIG. 6B, as in the case shown in FIG. 6A, the rear portion 12 is rotated along the side face of the bearing surface 11b from a right end position to a left end position of the bearing surface 11b shown in FIG. 6B. Then, the rear portion 12 reaches the stepped portion 11d to be moved from outside to inside of the bearing surface 11b, and engaged with the stepped portion 11d. As a result, the rear portion 12 is regulated by the stepped portion 11d, and the knife point portion 5 is prevented from being rotated again in a protruding direction (from left to right in the drawing).

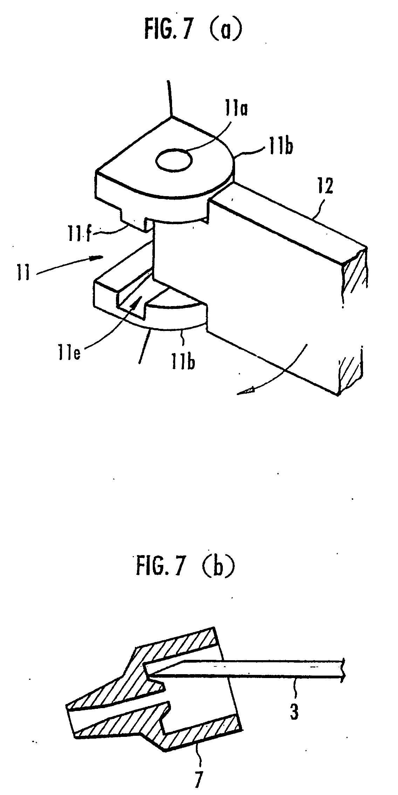

As shown in FIG. 7A, with the pair of upper and lower bearing surfaces 11b and 11b as rotation guiding members, the protrusion preventing means may be provided with a groove portion 11e in the lower bearing surface 11b, and a protruded portion 11f in the upper bearing surface 11b.

According to the constitution shown in FIG. 7A, when the front enveloping portion 10 and the rear portion 12 are expanded as shown in FIGS. 4 and 5, the rear portion 12 is dropped into the groove portion 11e, and prevented from being pulled out by the protruded portion 11f. As a result, the front enveloping portion 10 and the rear portion 12 are inclined obliquely downward with the hinge portion 11 as a basic point, inclining the support member 7 with respect to an extended direction of the inner needle 3 as shown in FIG. 7B. Thus, it is possible to prevent the knife point portion 5 of the inner needle 3 from being protruded again from the support member 7.

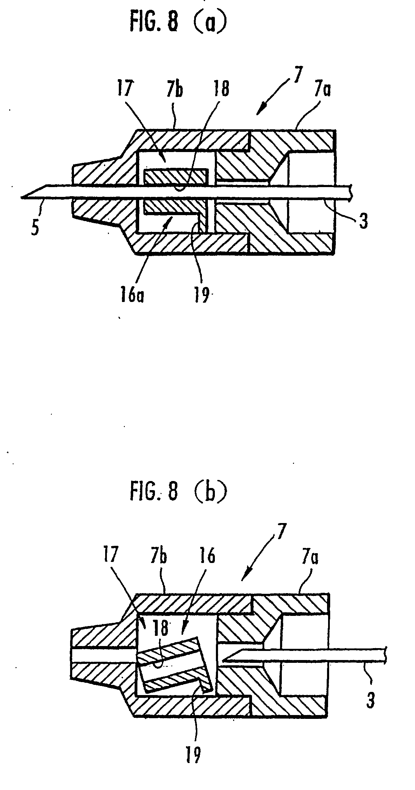

Further, as shown in FIG. 8A, the protrusion preventing means may be provided with a spacing member 16a in the support member 7. In this case, the support member 7 includes a housing portion 7a for housing the knife point portion 5 of the inner needle 3, and a support portion 7b fitted over a tip of the housing portion 7a to support the outer needle hub 4. When the support portion 7b is fitted over the tip of the housing portion 7a, a hollow chamber 17 is formed between it and the housing portion 7a, and the spacing member 16a is then provided in the hollow chamber 17. The spacing member 16a is a cylindrical body provided with a through-hole 18 for inserting the inner needle 3 and, on one end, a ground portion 19 to be grounded to an inner wall of the support portion 7b

According to the constitution shown in FIG. 8A, when the inner needle 3 is inserted from the housing portion 7a through the through-hole 18 into the support portion 7b, the through-hole 18 is in parallel with the extended direction of the inner needle 3. However, when the inner needle 3 is retreated and housed in the housing portion 7a, the spacing member 16a is only supported by the ground portion 19 on one end. Accordingly, as shown in FIG. 8B, the other end is brought into contact with the inner wall of the support portion 7b, and the through-hole 18 is inclined with respect to the extended direction of the inner needle 3. Therefore, it is possible to prevent the knife point portion 5 of the inner needle 3 from being protruded again from the support portion 7b.

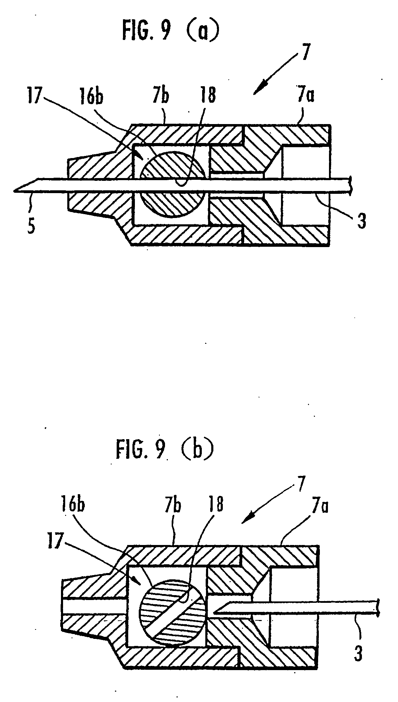

Further, in place of the cylindrical spacing member 16a shown in FIG. 8A, the protrusion preventing means may be provided with a spherical spacing member 16b as shown in FIG. 9B. The spacing member 16b includes a through-hole 18 on its spherical center. When the inner needle 3 is inserted from the housing portion 7a through the through-hole 18 into the support portion 7b, the through-hole 18 is in parallel with the extended direction of the inner needle 3. However, when the inner needle 3 is retreated and housed in the housing portion 7a, the spacing member 16b is rotated in an optional direction and, as shown in FIG. 9(b), the through-hole 18 is inclined with respect to the extended direction of the inner needle 3. Thus, it is possible to prevent the knife point portion 5 of the inner needle 3 from being protruded again from the support portion 7b.

Next, description will be made of a self-retaining needle 21 according to a second embodiment of the present invention.

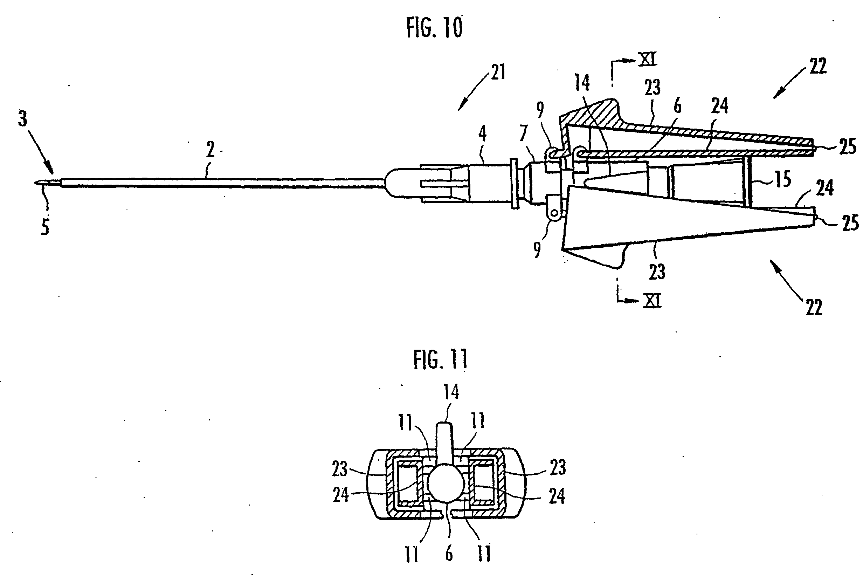

As shown in FIG. 10, the self-retaining needle 1 is completely similar in constitution to the self-retaining needle 1 of the first embodiment except the structure of the enveloping member 22. Thus, in the self-retaining needle 21, components similar to those of the self-retaining needle 1 are denoted by similar reference numerals, and detailed description thereof will be omitted.

The enveloping member 22 is designed to connect a support member 7 with an inner needle hub 6, and envelope an entire inner needle 3 when the inner needle 3 is retreated toward the rear of an outer needle hub 4. Accordingly, the enveloping member 22 is arranged to be divided along both side faces of the inner needle hub 6, and composed of a front enveloping portion 23 rotatably attached to a hinge portion 9 protruded in both sides from the support member 7, and a rear enveloping portion 24 rotatably attached to a hinge portion 11 protruded in both sides from the inner needle hub 6. The front enveloping portion 23 and the rear enveloping portion 24 are connected to each other through a thin portion 25 so as to be folded.

As shown in FIG. 11, the front enveloping portion 23 is formed in a tub shape, where a surface opposite the inner needle hub 6 is opened. On the other hand, the rear enveloping portion 24 is formed in a sub shape, where a surface opposite the opened surface of the front enveloping portion 23 is opened. In a state where a knife point portion 5 of the inner needle 3 is protruded from a tip of the outer needle 2; the front enveloping portion 23 and the rear enveloping portion 24 are folded at a thin portion 25. In the above state, the rear enveloping portion 24 is arranged between the front enveloping portion 23 and the inner needle hub 6, and housed in the tub-shaped front enveloping portion 23.

According to the self-retaining needle 21 shown in FIG. 10, by an operation similar to that of the self-retaining needle 1 of the first embodiment of FIG. 1, the knife point portion 5 of the inner needle 3 is thrust into a blood vessel or the like of a patient, and the outer needle 2 is introduced into the blood vessel or the like. At this time, in the self-retaining needle 21, the front and rear enveloping portions 23 and 24 of the enveloping member 22 are folded at the thin portion 25, and the rear enveloping portion 24 is housed in the tub-shaped front enveloping portion 23. Thus, the enveloping member 22 is prevented from interfering with the operation of sticking the inner needle 3 into the blood vessel or the like, and introducing the outer needle 2 into the blood vessel.

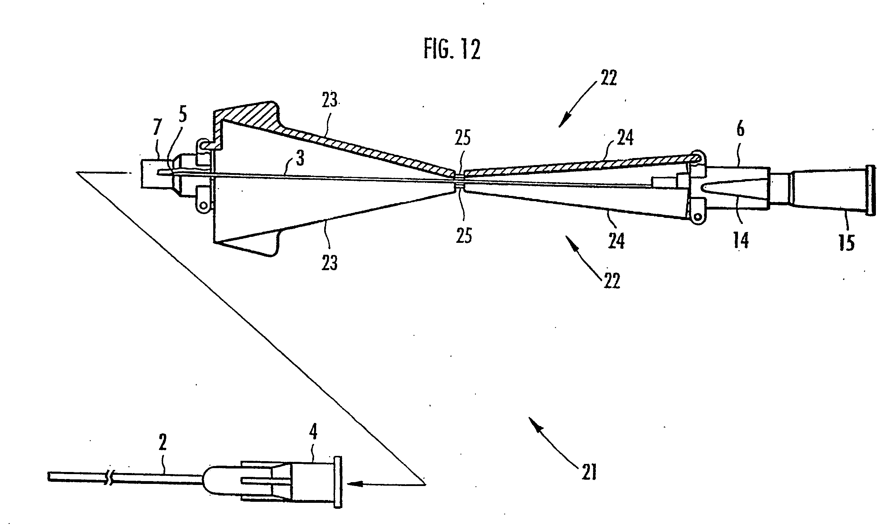

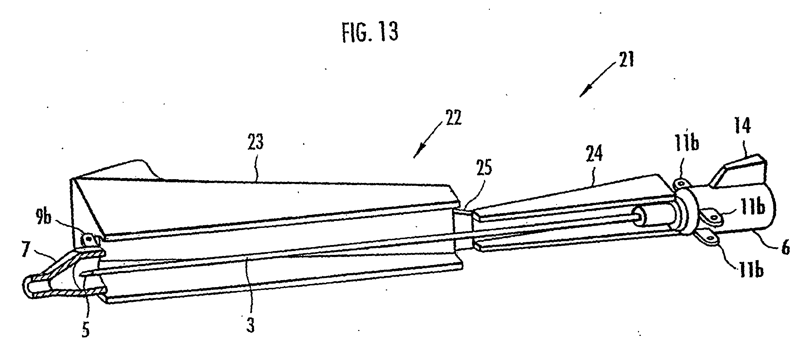

Then, a gripper 14 provided on the inner needle hub 6 is gripped to retreat the inner needle hub 6. Following the retreating, the front and rear enveloping portions 23 and 24 are rotated toward the outer needle hub 4 and, further as shown in FIG. 12, completely expanded along the inner needle 3 on both side faces of the same to be linear. Thus, when the support member 7 is removed from the outer needle hub 4, as shown in FIG. 13, the opened surfaces of the tub-shaped front and rear enveloping portions 23 and 24 face the inner needle 3. As a result, the entire inner needle 3 is enveloped by the front enveloping portions 23 and 23, and the rear enveloping portions 24 and 24 arranged on both sides of the inner needle 3. Thus, it is possible to surely prevent the medical personnel or the worker engaged in needle disposal from accidentally sticking the knife point portion of the inner needle 3, or touching the inner needle 3.

For explanation, only one front enveloping portion 23 and one rear enveloping portion 24 are shown in FIG. 13.

In this case, the self-retaining needle 21 comprises protrusion preventing means provided to prevent the knife point portion 5 of the inner needle 3 retreated into the support member 7 once after the outer needle hub 4 from being protruded again from the support member 7. The protrusion preventing means can be similar in constitution to that of the self-retaining needle 1 of the first embodiment.

In the first embodiment, the front enveloping portion 10 and the rear portion 12 are folded at the thin portion 13. In the second embodiment, the front and rear enveloping portions 23 and 24 are folded at the thin portion 25. However, the front enveloping portion 10, the rear portion 12, and the front and rear enveloping portions 23 and 24 may be folded through hinge portions made of mechanical components in place of the thin portions 13 and 25.

### Industrial Applicability

The present invention can be applied to a self-retaining needle as a medical instrument, which is connected to an infusion device or the like for introducing blood, medicine or the like into a body of a patient.

## Claims

1. A self-retaining needle having a hollow outer needle, an outer needle hub provided in a base of the outer needle, a hollow inner needle having a knife point portion on a tip and a length of the knife point portion protruded from a tip of the outer needle when it is inserted from the outer needle hub into the outer needle, and an inner needle hub provided in a base of the inner needle and positioned at the rear of the outer needle hub when the knife point portion of the inner needle is protruded from the tip of the outer needle, comprising:
a support member for detachably supporting the outer needle hub in front of the inner needle hub; and
an enveloping member for connecting the support member with the inner needle hub and enveloping at least a front of the inner needle when the inner needle is retreated toward the rear of the outer needle hub,
wherein 33a front portion and a rear portion of the enveloping member are connected so as to be freely folded; when the knife point portion of the inner needle is protruded from the tip of the outer needle, the front portion and the rear portion of the enveloping member are folded, so that the rear portion is housed between the inner needle hub and the front portion; and when the inner needle is retreated toward the rear of the outer needle hub, the front portion and the rear portion thereof are expanded along both side faces of the inner needle.

2. The self-retaining needle according to claim 1, wherein the front portion is rotatably attached to the support member at an end, the rear portion is rotatably attached to the inner needle hub at a rear end, and a rear end of the front portion and a tip of the rear portion are folded through a hinge portion.

3. The self-retaining needle according to claim 1 or 2, wherein when the inner needle is retreated toward the rear of the outer needle hub, and the front portion and the rear portion are expanded along both side faces of the inner needle, the enveloping member envelops a front of the inner needle by the front portion.

4. The self-retaining needle according to claim 3, wherein the front portion is formed in a tub shape, having a surface opposite the inner needle hub opened, the rear portion is formed in a flat plate shape, and the rear portion is housed in the tub-shaped front portion while the front portion and the rear portion are folded.

5. The self-retaining needle according to claim 1 or 2, wherein when the inner needle is retreated toward the rear of the outer needle hub, and the front portion and the rear portion are expanded along both side faces of the inner needle, the enveloping member envelops the inner needle by the front and rear portions.

6. The self-retaining needle according to claim 5, wherein the front portion is formed in a tub shape having a surface opposite the inner needle hub opened, the rear portion is formed in a tub shape having a surface opposite the opened surface of the front portion opened, and the rear portion is housed in the tub-shaped front portion while the front portion and the rear portion are folded.

7. The self-retaining needle according to any one of claims 1 to 6, further comprising protrusion preventing means for preventing the knife point portion of the inner needle from being protruded again when the inner needle is retreated toward the rear of the outer needle hub, and the front portion and the rear portion are expanded along both side faces of the inner needle.

8. The self-retaining needle according to claim 7, wherein the protrusion preventing means includes a rotation guiding member, in which the rear portion of the enveloping member is rotatably attached to the inner needle hub, and the inner needle hub guides rotation of the rear portion, a slope portion formed to be gradually protruded from a surface of the guiding member, and a stepped portion provided on a boundary with the surface of the guiding member on a most protruded part of the slope portion, and the rear portion is positioned on a base of the slope portion in a folded state, guided and rotated by the slope portion following a laying-out operation, and engaged with the stepped portion by going over the most protruded part of the slope portion to prevent the knife point portion of the inner needle from being rotated again in a protruding direction.

9. The self-retaining needle according to claim 7, wherein the protrusion preventing means includes a rotation guiding member, in which the rear portion of the enveloping member is rotatably attached to the inner needle hub, and the inner needle hub guides rotation of the rear portion, and a stepped portion formed on a side face of the rotation guiding member from outside to inside, the rear portion is rotated along the side face of the rotation guiding member following a laying-out operation from a folded state, and engaged with the stepped portion by moving from outside to inside on the side face of the rotation guiding member to prevent the knife point portion of the inner needle from being rotated again in a protruding direction.

10. The self-retaining needle according to claim 7, wherein the protrusion preventing means includes a pair of upper and lower rotation guiding members, in which the rear portion of the enveloping member is rotatably attached to the inner needle hub, and the inner needle hub guides rotation of the rear portion, a groove portion provided on a surface of one rotation guiding member, and a protruded portion provided on a surface of the other rotation guiding member to face the groove portion, and the rear portion is guided and rotated by the rotation guiding member following a laying-out operation from a folded state, dropped into the groove portion, and prevented from being pulled out from the groove portion by the protruded portion, and an extended direction of the inner needle is inclined with respect to the support member.

11. The self-retaining needle according to claim 7, wherein the protrusion preventing means includes a hollow chamber provided in the support member, and a spacing member provided with a through-hole for inserting the inner needle, and moved in the hollow chamber to incline the through-hole with respect to an extended direction of the inner needle when the inner needle is pulled out from the through-hole.
